# EUROPEAN PATENT SPECIFICATION

(11) **EP 0 477 386 B1**
(45) Date of publication and mention of the grant of the patent: **23.07.1997**
(21) Application number: 91907509.3
(22) Date of filing: 12.04.1991
(51) Int. Cl.: A61K 9/12, A61K 9/14, A61K 9/51, A61K 9/72, A61K 38/21

(54) **PHARMACEUTICAL AEROSOL FORMULATION OF SOLID POLYPEPTIDE MICROPARTICLES AND METHOD FOR THE PREPARATION THEREOF**
AEROSOLFORMULIERUNG FESTE POLYPEPTID MIKROTEILCHEN ENTHALTEND UND VERFAHREN ZU IHRER HERSTELLUNG
FORMULATION D'UN AEROSOL A USAGE PHARMACEUTIQUE COMPORTANT DES MICROPARTICULES SOLIDES DE POLYPEPTIDE ET PROCEDE DE PREPARATION

(30) Priority: 13.04.1990 JP 98353/90
(43) Date of publication of application: 01.04.1992
(73) Proprietor: TORAY INDUSTRIES, INC., Tokyo 103 (JP)
(72) Inventor: PLATZ, Robert, M., Half Moon Bay, CA 94019 (US); UTSUMI, Jun, N-104, 1-31-17, Tsunishi, Kanagawa 248 (JP); SATOH, Yuichiro, 4-9-5, Kugahara, Tokyo 146 (JP); NARUSE, Norio, 1-24-15 Tsunishi, Kanagawa 248 (JP)
(74) Representative: Kador & Partner
(86) International application number: JP9100486
(87) International publication number: WO9116038

(56) References cited:
- EP-A- 0 122 036
- EP-A- 0 215 658
- EP-A- 0 257 956
- EP-A- 0 289 336
- EP-A- 0 396 903
- WO-A-89/05158

## Description

### FIELD OF THE INVENTION

The present invention relates to a novel pharmaceutical aerosol formulation of solid polypeptide microparticles and to a method for preparing the same formulation.

### BACKGROUND OF THE INVENTION

In recent years, many biologically active polypeptides have been tried for pharmaceutical use. The polypeptides as pharmaceutical proteins have been required to have high therapeutic efficacy with less adverse effects and high stability. Regard to the formulation, the polypeptides are generally administered as aqueous solution because they are enzymatically degraded in the digestive tract, and they are poorly absorbed by the biological membranes in other parts of the body because of their molecular size and enzymatic degradation in transit to the circulation. For the polypeptide administration, intravenous, intramuscular or subcutaneous injections are usual routes, while neither oral administration nor transdermal administration is not established. An improved method for the polypeptide administration has therefore been required to decrease the complexity of handling and the physical burden of the patient.

Inhalation is a potent desirable method for the polypeptide administration. An aerosol having aqueous droplets is produced by nebulization of aqueous solution with a jet nebulizer. On the other hand, an aerosol having solid particles is generated with either a metered-dose inhaler or a dry-powder inhaler, both of which are used for β₂-adrenergic agonists, steroids and antiallergics to treat asthma.

Particle size is a key factor to control the deposition region of the particles in respiratory tract. Small particles capable of reaching to alveoli must be 0.5 to 7 µm in diameter (Porush et al., J. Amer, Pharm. Ass. Sci. Ed., 49, 70, 1960), preferably less than 5 µm (Newman and Clarks, Thorax, 38, 881, 1983).

Aerosol formulations of polypeptides have recently been developed such as a solid particle form of insulin (Lee and Sciarra, J. Pharm. Sci., 65, 567, 1976), and recombinant αl-antitrypsin (Hubbard et al, Proc. Natl. Acad. Sci. U.S.A., 86, 680, 1989). In these trials, the diameter of aerosol particle is suitably less than 5 µm.

While, for intranasal administration of the polypeptide, the larger particle size is required both in the aqueous and solid aerosols. For instance, aqueous spray of the human leukocyte IFN was applied to prevent from Rhino virus infection (Scott et al., Br. Med. J., 284, 1822, 1982) and the polypeptide formulation for nasal administration was described in Japanese patent publication (KOKOKU) No. 62-37016 wherein the median particle size is limited to between 10 to 250 µm in diameter to refrain from the particle deposition into lung. EP 257956 describes a device and dispersion for intrapulmonary delivery of peptide growth factors and cytokines, such as somatropin, somatrem, interleukin (IL)-1 or -2, tumor necrosis factor (TNF)-α or -β plus interferon (IFN), or a combination of IFN and IL-2. However, IFN alone, ILs-6, -8 and -11, antibody, antibody fragments, and immunoconjugate referred to by the present invention are not disclosed in this prior publication.

Maitani et al., (Drug Design and Delivery, 1, 65, 1986) and Maitani et al., (ibid., 4, 109, 1989) have developed IFN-β formulations for nasal administration that use enhancers to facilitate absorption across the nasal mucosa. However, even with permeation enhancers, the bioavailability was too low for a pharmaceutically acceptable product.

To deliver the polypeptide particles efficiently, it is necessary not only to control the particle size but also to prepare the formulation with high efficiency.
Therefore, optimal conditions must be determined for each peptide including aqueous or solid state to prepare the preferred aerosol formulation.

Most recently, pulmonary administration of proteins and other macromolecules by aerosol to produce a systemic effect has been reported. Recombination forms of the IFN-γ and tumor necrosis factor (Debs et al., J.Immunol., 140, 3482, 1988) and human interferon α (Kinnula et al., J.Interferon Res., 9, 419, 1989) have been observed in the bloodstream after aerosol administration to the lung. Recombinant human growth hormone (HGH) has been shown to be rapidly absorbed from the lung when delivered by aqueous aerosol and the aerosol HGH promoted the growth in test animals like that obtained with subcutaneous injection (Fuchs et al., J. Clin. Invest., submitted).

In these cases, the aerosol was produced from an aqueous solution containing the proteins using a jet nebulizer. However, in the case of IFN-β, the protein is particularly unstable in the aqueous solution and to the recirculation action in the jet nebulizer, and the type of device commonly used to produce aerosols from medicated solutions promotes molecular aggregation of the IFN-β rendering the protein biologically inactive.

### SUMMARY OF THE INVENTION

The present invention relates to a novel pharmaceutical aerosol formulation of solid polypeptide microparticles. The formulation according to the invention comprises solid micronized particles of biologically active human interferon beta, the particle size thereof being of 0.5 to 10 µm in median diameter. Further, the micronized particle contains human serum albumin and sugar or sugar alcohol for ensuring the stability of the polypeptide, and in addition the produced formulation may further comprise surfactant for improving the dispersion of the particles. The polypeptide may be prepared as a dry powder which can be milled to an appropriate particle size to target either an upper or lower respiratory tract. The polypeptide aerosol suspension prepared in a pressurized metered-dose inhaler and administered by oral inhalation was found to produce desired local and systemic effects in animal tests. The biological response obtained with the polypeptide aerosol compares favorably to the response obtained from parenteral administration.

### BRIEF DESCRIPTION OF THE DRAWINGS

Fig. 1 shows the effect of grinding pressure on the median diameter of micronized lyophilized human serum albumin (HSA).

Fig. 2 shows the IFN-β deposition in respiratory tract after IFN-MDI inhalation to rabbit.

Fig. 3 shows the IFN-β serum concentration after inhalation of IFN-MDI to rabbit.

### DETAILED DESCRIPTION OF THE INVENTION

The aerosol formulation has a great advantage in being easy to administer the formulated drug. However, since many biologically active polypeptides have their own intrinsic properties, general methods to formulate them in a stable form are not established yet. For example, some polypeptides lose their biological activities by oxidation, aggregation and autolysis in the aqueous solution. The nebulization of the polypeptide by a jet nebulizer in aqueous conditions may increase the loss of the biological activity by the shearing stress during the atomization of the solution. With respect to the aerosol formulation, the solid state is more suitable to maintain the biological activity.

The present invention provides a solid polypeptide aerosol formulation to deliver to the respiratory tract, the polypeptide being human IFN beta.

Polypeptides may be obtained from natural sources, recombinant microorganisms (e.g., Escherichia coli and yeast), recombinant insect cells (e.g., silk worm), recombinant mammalian cells (e.g., chinese hamster ovary cells, murine C127 cell and simian COS cell) and chemically synthesized products. The polypeptide is extracted and purified in aqueous conditions up to guaranteed pharmaceutical grade. In the case of a labile polypeptide, human serum albumin (HSA) may be added to the purified preparation (more than 0.5% (w/w)), and some sugars or sugar alcohols may also be added to the preparation, if necessary. The symbol (w/w) refers to the weight of the ingredient per weight of the formulation.

Sugar or sugar alcohol is selected at least one from lactose, maltose, sorbitose, tolehalose, xylose, mannitol, sorbitol and xylitol. The sugar or sugar alcohol is added to the polypeptide preparation in the range of 0.01 to 200% (w/w), preferably 1 to 50% (w/w). The mixture is lyophilized followed by milling.

The particle size is controlled by grinding pressure in the milling process. The grinding pressure is adjusted in the range of 5 to 120 psig, preferably 5 to 15 psig for large particles (4.0 to 10 µm in diameter) and 15 to 100 psig for small particles (0.5 to 4.0 µm).

The milling produces micronized solid particles between 0.5 to 4.0 µm in median diameter for intrapulmonary administration and the other particles of less than 10 µm in median diameter for nasal and upper respiratory tract administrations. The size distribution of the particles is defined by more than 50% of total particles, preferably more than 75%. The particle size of the powder in suspension is determined by centrifugal sedimentation particle analyzer model CAPA-700 (Horiba Ltd., Kyoto, Japan) or by QCM-Cascade Impactor Model PC-2 (California Measurement Inc., CA, U.S.A.) for the powder dispersed in an aerosol.

To improve the dispersion in liquid suspension and to inhibit the aggregation, some kind of surfactant is preferably added to the milled polypeptide powder. The surfactant includes nonionic surfactants such as mono and diglycerides, sorbitan fatty acid esters and polyoxyethylene acids and polyoxyethylene alcohols, and amphoteric surfactants, phospholipids, phosphatidylcholine and natural lecithins. The surfactant is preferably selected from at least one of sorbitan trioleate (SPAN 85), soya lecithin, oleyl alcohol or polyoxyethylene (POE) hydrogenated castor oil in the range between 0.05 to 2% (w/w).

The polypeptide formulation may also contain some mineral ions, calcium ion, magnesium ion, sodium ion, potassium ion and chlorine ion, to maintain the desired physiological osmolarity. Moreover, a biological surfactant derived from alveolus may also be added to the polypeptide formulation to decrease mucosal stimulation, if necessary.

The polypeptide formulation for inhalation is dispersed by volatilization of a liquid propellant or the shear force in a high velocity gas stream. Generally, the polypeptide particles are packed in an air-tight vessel and sprayed out by compressed air, compressed carbon dioxide, or fluorocarbon propellants. Examples of the fluorocarbon propellants include chlorofluorocarbons (CFC-11, CFC-12 and CFC-114), hydrochlorofluorocarbons (HCFC-123, HCFC-124 and HCFC-141) and hydrofluorocarbons (HFC-125 and HFC-134a).

The polypeptide formulation disclosed in the present specification is particularly valuable for a polypeptide which is unstable in aqueous solution, such as IFN-β.

The IFN-β can be formulated either as a dry powder suspended in propellants and administered as a pressurized metered-dose aerosol, or as a dry powder contained in a capsule on a blister pack that is administered with a dry powder inhaler. The IFN-β exists in a solid form in both of these formulations and the protein is considerably more stable when prepared as a lyophilized powder than in solution. These novel aerosol formulations, in which IFN-β is prepared as a dried solid, overcome the problems with instability of the protein in a jet nebulizer and enables IFN-β to be delivered as an aerosol. This invention provides a convenient noninvasive means of administering therapeutically meaningful dosages of interferon.

The suspension aerosol formulation contemplated in this invention is administered with a metered-dose inhaler. Specific examples of commercial instruments that utilize this type of device for the delivery of the peptide for local treatment of lung disease include Ventolin Inhaler (Glaxo, Inc., Research Triangle Park, NC, U.S.A.) and Intal Inhaler (Fisons, Corp., Bedford, MA, U.S.A.). The dry powder formulation contemplated in this invention is administered with a dry-powder inhaler. Commercial dry-powder inhalers include Ventolin Rotahaler (Glaxo, Inc., Research Triangle Park, NC, U.S.A.) and Spinhaler (Fisons Corp., Bedford, MA, U.S.A.).

### EXAMPLES

The present invention will hereinafter be further illustrated by the following examples.

### EXAMPLE 1: Preparation of micronized IFN-β

It has been found that the excipients preserve the IFN-β activity during preparation of the dry powder formulation. Aqueous IFN-β formulations containing human serum albumin (HSA), sodium chloride, and sorbitol were prepared having the compositions listed in Table 1.

**Table 1**

| IFN-β preparations for lyophilyzation | | |
|---|---|---|
| Component | Solution A | Solution B |
| IFN-β | 15 µg/ml | 15 µg/ml |
| Human serum albumin | 1.5 mg/ml | 15 mg/ml |
| NaCl | 0.6 mg/ml | 0.6 mg/ml |
| D-sorbitol | - | 4.5 mg/ml |
| Total solids | 2.1 mg/ml | 20.1 mg/ml |

The solutions were lyophilized in a freeze dryer (Model No. GT4, Leybold Colonge, Germany) and the dried powder was subsequently milled in a jet mill (Sturtevant, Boston, MA, U.S.A.). The lyophilized and micronized powder was assayed with using an enzyme immunoassay (EIA) (Yamazaki et al., J. Immunoassay, 10, 57, 1989). This EIA is specific for the IFN-β monomer, biological active form of the protein.

Table 2 contains the ratio of the IFN-β activity in the powder sample to the activity of the original solution. Values in Table 2 are reported as the ratio ± standard deviation of two measurements of the IFN activity in the sample powder to the activity of the original solution multiplied by 100. The IFN-β powder was reconstituted in Eagle's minimum essential medium (MEM) supplemented with 10% fetal calf serum (FCS). The IFN activity was retained in Formulations A and B more than 65% activity during lyophilization and milling steps in preparing the micronized powder. These conditions are acceptable for the preparation of micronized powder.

**Table 2**

| Fraction of IFN-β activity retained in preparing the micronized powder | | | |
|---|---|---|---|
| Formulation | IFN-β Activity of Lyophilized Powder | IFN-β Activity of Milled Powder | Particle Size |
| A | 107 ± 17% | 65 ± 4.8% | 2.3 ± 1.7 µm |
| B | 110 ± 25% | 102 ± 3.8% | 1.6 ± 1.2 µm |

### EXAMPLE 2: Particle size control of micronization

A jet mill (Sturtevant) was used to prepare micronized particles containing IFN. Dry nitrogen supplied from liquid dewars and filtrated with ultrahigh efficiency filters (Millipore Corp., Bedford, MA, U.S.A.) was used to minimize contamination of the IFN powder during milling. The jet mill was operated to prepare a powder having greater than 50% of the particle less than 3 µm in diameter and 90% of the particle less than 5 µm in diameter. The fine particles are required for efficient deposition in the pulmonary region of the respiratory tract where macromolecules are thought to be absorbed across the epithelia, while the coarser particles deposit in the pharyngal region and the upper respiratory tract.

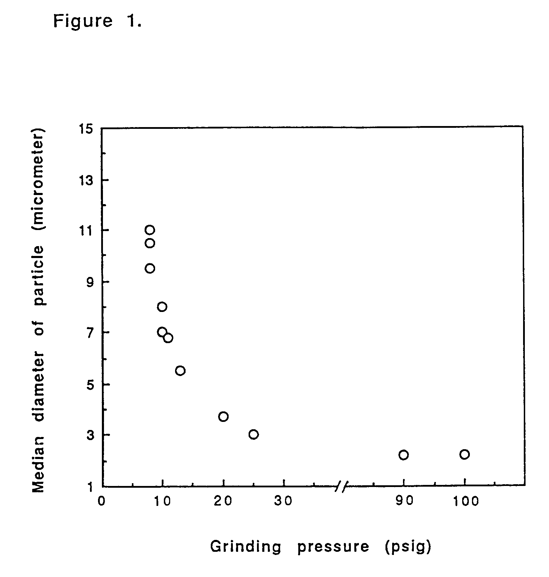

The HSA powder as a test sample was micronized under various grinding pressures. The particle size was measured by Horiba particle analyzer model CAPA-700. As shown in Figure 1, the median particle size of the micronized powder is dependent on the grinding pressure of the jet mill. To obtain fine particles less than 4 µm in diameter, the grinding pressure must be 15 to 100 psig. On the other hand, coarser particles could be prepared by operating mill at a low pressures (5 to 15 psig). These larger particles had a median particle diameter of between 5 to 15 µm which would deposit primarily in the nasal and pharyngal regions and upper respiratory tract when administered nasally as a pressured metered-dose aerosol.

### EXAMPLE 3: Compatibility of IFN-β with metered-dose inhaler (MDI) aerosol excipients

A series of samples was prepared to determine whether the aerosol excipients are compatible with IFN-β. Four mg of micronized powder prepared by milling under 100 psig of grinding pressure, a lyophilized powder prepared from Formulation B, were weighed into small vial. A surfactant at a final concentration of either 0.1% (w/w) or 1.0% (w/w) and a propellant (CFC-12) were added to the vial . The more hydrophilic surfactants were dissolved in a solvent (CFC-11). The samples were then chilled in dry ice and set at room temperature for 2 to 7 days, after which the vials were opened and the propellant and the solvent allowed to evaporate. The remaining nonvolatiles, powder and surfactant were reconstituted in 5 ml of either Eagle's MEM with 10% FCS and the activity of the IFN-β in the sample was assayed. The measured sample activities were compared with the expected activity in the sample which was based on the activity of the micronized powder.

Table 3 contains the results of the assay for a variety of surfactants suitable for dispersion of the powder in suspension. It was discovered that the propellants CFC-12 and CFC-11 along with the surfactants of sorbitan trioleate, oleyl alcohol, POE hydrogenated castor oil and soya lecithin did not adversely affect the IFN-β activity. These surfactants in the concentrations of 1.0% (w/w) resulted in a loss of IFN-β activity in samples prepared from Formulation B. The lower activity was quite probably a result of the surfactants retarding the dissolution of the micronized powder in the resuspending medium. The IFN-β activity was retained in the samples prepared with powder Formulation B; the sorbitan trioleate may promote the dissolution of the IFN-β as a soluble monomer.

**Table 3**

| Compatibility of IFN-β with MDI aerosol excipients | | | |
|---|---|---|---|
| Surfactant | Propellant CFC-12/11 blend | 0.1% Surfactant | 1.0% Surfactant |
| Oleic acid | 100/0 | 23% | 26% |
| Oleyl alcohol | 100/0 | 101% | 73% |
| Sorbitan trioleate | 100/0 | 92% | 29% |
| Aerosol AT | 100/0 | 27% | 31% |
| Soya lecithin | 90/10 | 98% | 53% |
| POE hydrogenated castor oil | 90/10 | 93% | 87% |
| Cetyl pyridinium chloride | 90/10 | 25% | - |
| (Without surfactant | | 102%) | - |

### EXAMPLE 4 (Comparison): Compatibility test in aqueous solution

To compare the compatibility of surfactants between the solid and aqueous states, Formulation B was dissolved in Eagle's MEM with 10% FCS and 0.1% (w/w) each surfactant. After the incubation at room temperature for 48 hours, IFN-β activity was assayed by EIA.

The results are shown in Table 4, where residual IFN-β activities under the existence of each surfactant were much lower than those in the solid state in Table 3. This indicates that the solid state of IFN-β is more stable to the surfactant than the aqueous state.

**Table 4**

| Compatibility of IFN-β in aqueous solution | |
|---|---|
| Surfactant | 0.1% Surfactant |
| Oleyl alcohol | 40% |
| Sorbitan trioleate | 57% |
| Soya lecithin | 16% |
| POE hydrogenated castor oil | 26% |
| (Without surfactant | 57%) |

### EXAMPLE 5: MDI formulation of IFN-β

The MDI formulations of IFN-β were prepared by using four kinds of surfactants, oleyl alcohol (OA), sorbitan trioleate (SPAN85), Soya lecithin (SL) and POE hydrogenated castor oil (HCO) at the concentration of 0.1% (W/W). MDI samples were prepared according to the procedures in EXAMPLE 3. Preparative yields were summarized in Table 5. Contents of Formulations A and B are described in Table 1.

**Table 5**

| Preparation of IFN-MDI using four surfactants | | | |
|---|---|---|---|
| Step | | Preparative Yield (Step%/Overall%) | |
| | | Formulation A | Formulation B |
| Starting Solution | | 100/100 | 100/100 |
| Lyophilization | | 107/107 | 74/74 |
| Milling | | 65/70 | 101/75 |
| MDI Powder | -OA | 96/68 | 78/58 |
| | -SPAN85 | 81/56 | - |
| | -SL | 68/47 | 100/75 |
| | -HCO | 84/58 | - |

All the IFN-MDI preparations using the four different surfactants gave the final preparative yield more than 50%. These conditions are acceptable for the preparation of MDI. Moreover, the IFN-β activity in MDI formation was stable at 4°C at least for one year.

### EXAMPLE 6: Deposition of IFN-β in lung after the inhalation of IFN-MDI to rabbit

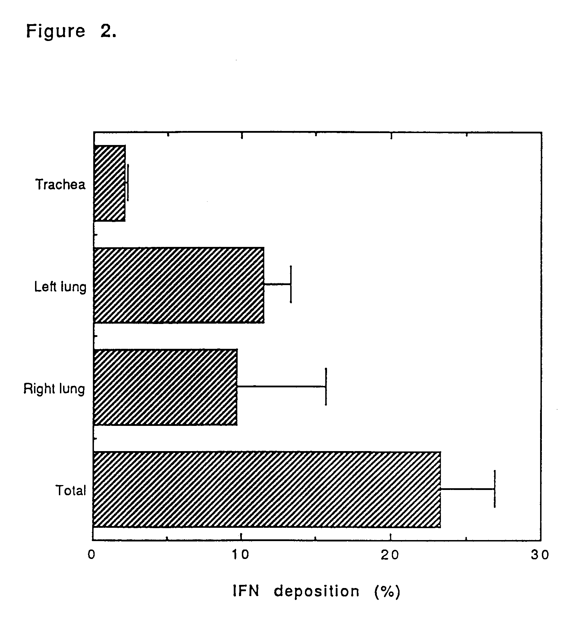

The IFN-MDI samples were prepared according to EXAMPLE 5. Three New Zealand White rabbits between 2.9 and 3.2 Kg were used. A rabbit was mildly restrained in an animal chamber and attached a mouthpiece connected with a cylindrical spacer (300 ml of space volume) covering nose and mouth. Solid IFN-β particles from OA-IFN-MDI were puffed into the spacer. In dosing the rabbit, 20 accutuations of the MDI (2 x 10⁶ IU/Kg) were administered by breathing in approximately 15 minutes. At 15 minutes after the inhalation of the MDI, the rabbit was sacrificed with a 50mg/Kg injection of sodium thiopental and trachea and whole lung were removed. These tissues were homogenized in Eagle's MEM supplemented with 10% FCS and the IFN-β activity in the supernatant was assayed by EIA. As shown in Figure 2, the IFN-β was found to deposit in all regions of the respiratory tract. Total deposition ratio was 23.0 ± 3.6% to the inhaled dose.

### EXAMPLE 7: Serum titer of IFN-β after inhalation of IFN-MDI to rabbit

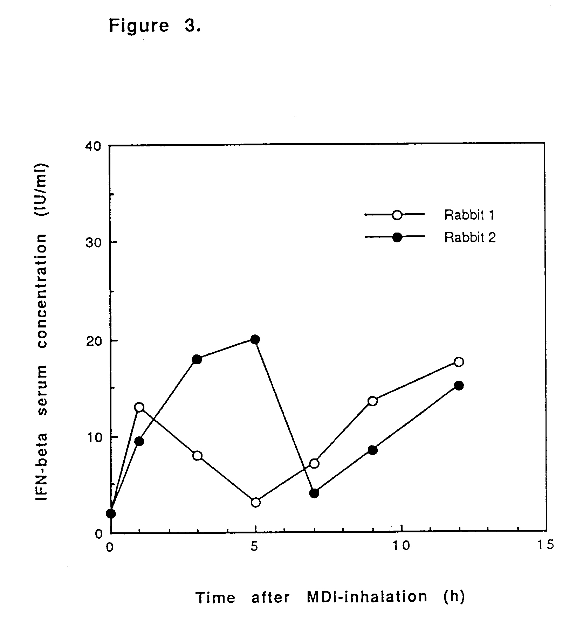

Two rabbits were received IFN-β by inhalation of SPAN85-IFN-MDI with 2 x 10⁶ IU/body according to the procedures in EXAMPLE 6. After the inhalation, blood was sampled from marginal ear vein. The IFN-β titer in the serum was assayed by EIA. The serum concentration-time profiles of IFN-β was illustrated in Figure 3. The IFN-β was significantly detected in sera of all rabbits.

### EXAMPLE 8: 2'5'-oligoadenylate synthetase (2'5'-AS) induction in rabbit liver by inhalation of IFN-MDI

The IFN-β was administered by the inhalation of SPAN85-IFN-MDI to ten rabbits with 2 x 10⁶ IU/Kg/day for five days according to the procedures in EXAMPLE 6. At day 6, the rabbits were sacrificed and their livers were removed. The liver samples were homogenized with 10mM HEPES buffer (pH 7.5) containing 10% glycerol and 0.1% Nonidet P-40. A specific enzyme induced by IFN, 2'5'-AS, was assayed by the method of Stark et al. (Methods Enzymol., 79, 194, 1981). As shown in Table 6, 2'5'-AS elevation was significantly observed in livers of inhalated rabbits.

**Table 6**

| 2'5'-AS elevation in livers of rabbits after the inhalation of IFN-MDI | | | |
|---|---|---|---|
| Condition | Total No. | 2'5'-AS Induction | |
| | | Negative | Positive |
| Control | 12 | 10 | 2 |
| Inhalated | 10 | 5 | 5 p<0.05* |

| | | | |
|---|---|---|---|
| *Statistical significance of X²-test. | | | |

## Claims

1. A pharmaceutical aerosol formulation comprising solid micronized particles of a biologically active human interferon beta, human serum albumin and a sugar or a sugar alcohol, wherein the median size of the particles is in the range of 0.5 to 10 µm.

2. The formulation of claim 1, further including a surfactant.

3. The formulation of claim 2, wherein the surfactant is selected from sorbitan trioleate, soya lecithin, oleyl alcohol, polyoxyethylene hydrogenated castor oil, and mixtures thereof.

4. The formulation of claim 1 which is effective to deliver the polypeptide to the lung, wherein the median size of the particles is in the range of 0.5 to 4.0 µm.

5. The formulation of claim 1 which is effective to deliver the polypeptide to the pharynx and upper respiratory tract, wherein the median size of the particles is in the range of 4.0 to 10 µm.

6. A method for preparing a pharmaceutical aerosol formulation as defined in claim 1, comprising lyophilizing an aqueous solution of the biologically active polypeptide and then milling the resultant dried powder into micronized particles under a grinding pressure of 1.35 to 9.25 bar (5 to 120 psig).

7. The method of claim 6, wherein the grinding pressure is 1 to 7.9 bar (15 to 100 psig).

8. The method of claim 6 wherein the median diameter of the micronized particles is in the range of 0.5 to 10 µm.

9. The method of claim, 6 wherein human serum albumin, a sugar, or a sugar alcohol is added to the aqueous solution of the biologically active polypeptide prior to lyophilization.

10. The method of claim, 6 wherein a surfactant is added to the micronized particles after milling.

11. The method of claim 10, wherein the surfactant is selected from sorbitan trioleate, soya lecithin, oleyl alcohol and polyoxyethylene hydrogenated castor oil.

## Patentansprüche

1. Pharmazeutische Aerosolformulierung, die feste mikronisierte Teilchen von biologisch aktivem Human-Interferon β, Human-Serumalbumin und Zucker oder Zuckeralkohol enthält, wobei die mittlere Größe der Teilchen im Bereich von 0,5 bis 10 µm liegt.

2. Formulierung nach Anspruch 1, die außerdem ein oberflächenaktives Mittel einschließt.

3. Formulierung nach Anspruch 2, wobei das oberflächenaktive Mittel aus Sorbitantrioleat, Sojalecithin, Oleylalkohol, Polyoxyethylen-hydriertem Castoröl und Mischungen davon ausgewählt ist.

4. Formulierung nach Anspruch 1, die der Lunge Polypeptid zuführen kann, wobei die mittlere Größe der Teilchen im Bereich von 0,5 bis 4,0 µm liegt.

5. Formulierung nach Anspruch 1, die dem Pharynx und dem oberen Atmungswegen Polypeptid zuführen kann, wobei die mittlere Größe der Teilchen im Bereich von 4,0 bis 10 µm liegt.

6. Verfahren zur Herstellung einer pharmazeutischen Aerosolformulierung nach Anspruch 1, die das Gefriertrocknen einer wäßrigen Lösung eines biologisch aktiven Polypeptids und das anschließende Mahlen des entstandenen getrockneten Pulvers zu mikronisierten Teilchen bei einem Mahldruck von 1,35 bis 9,25 bar (5 bis 120 psig) umfaßt.

7. Verfahren nach Anspruch 6, wobei der Mahldruck 1 bis 7,9 bar (15 bis 100 psig) beträgt.

8. Verfahren nach Anspruch 6, wobei der mittlere Durchmesser der mikronisierten Teilchen im Bereich von 0,5 bis 10 µm liegt.

9. Verfahren nach Anspruch 6, wobei der wäßrigen Lösung des biologisch aktiven Polypetids vor dem Gefriertrocknen Human-Serumalbumin, Zucker oder Zuckeralkohol zugesetzt werden.

10. Verfahren nach Anspruch 6, wobei den mikronisierten Teilchen nach dem Mahlen ein oberflächenaktives Mittel zugesetzt wird.

11. Verfahren nach Anspruch 10, wobei das oberflächenaktive Mittel aus Sorbitantrioleat, Sojalecithin, Oleylalkohol und Polyoxyethylen-hydriertem Castoröl ausgewählt ist.

## Revendications

1. Une formulation pharmaceutique pour aérosol, comprenant des particules solides micronisées d'un interféron bêta humain biologiquement actif, de la sérumalbumine humaine et un sucre ou un sucre alcool, dans laquelle la taille moyenne des particules est comprise entre 0,5 et 10 µm.

2. Une formulation selon la revendication 1, contenant de plus un tensioactif.

3. Une formulation selon la revendication 2, dans laquelle le tensioactif est choisi parmi le trioléate de sorbitan, la lécithine de soja, l'alcool oléylique, l'huile de ricin hydrogénée polyoxyéthylénée et leurs mélanges.

4. Une formulation selon la revendication 1, qui est efficace pour délivrer le polypeptide au niveau des poumons, dans laquelle la taille moyenne des particules est comprise entre 0,5 et 4 µm.

5. Une formulation selon la revendication 1, qui est efficace pour délivrer le polypeptide au niveau du larynx et de l'appareil respiratoire supérieur, dans laquelle la taille moyenne des particules est comprise entre 4,0 et 10 µm.

6. Un procédé pour préparer une formulation pharmaceutique pour aérosol telle que définie dans la revendication 1, comprenant la lyophilisation d'une solution aqueuse du polypeptide biologiquement actif et ensuite la pulvérisation de la poudre séchée résultante en particules micronisées, sous une pression de broyage de 1,35 à 9,25 bars (5 à 120 psig).

7. Un procédé selon la revendication 6, dans lequel la pression de broyage est comprise entre 1 et 7,9 bars (15 à 100 psig).

8. Un procédé selon la revendication 6, dans lequel le diamètre moyen des particules micronisées est compris entre 0,5 et 10 µm.

9. Un procédé selon la revendication 6, dans lequel on ajoute une sérumalbumine humaine, un sucre ou un sucre-alcool, à la solution aqueuse du polypeptide biologiquement actif avant la lyophilisation.

10. Un procédé selon la revendication 6, dans lequel on ajoute un tensioactif aux particules micronisées, après la pulvérisation.

11. Un procédé selon la revendication 10, dans lequel le tensioactif est choisi parmi le trioléate de sorbitan, la lécithine de soja, l'alcool oléylique et l'huile de ricin hydrogénée polyoxyéthylénée.
